# EUROPÄISCHE PATENTSCHRIFT

(11) **EP 2 536 634 B1**
(45) Veröffentlichungstag und Bekanntmachung des Hinweises auf die Patenterteilung: **02.12.2015**
(21) Anmeldenummer: 11711258.1
(22) Anmeldetag: 05.02.2011
(51) Int. Cl.: B64G 1/60, A61F 5/37

(54) **VORRICHTUNG ZUR FIXIERUNG EINER TESTPERSON AUF EINER STANDFLÄCHE**
DEVICE FOR FIXING A TEST PERSON ON A STANDING SURFACE
DISPOSITIF PERMETTANT DE FIXER SUR UNE SURFACE D'APPUI UNE PERSONNE VOLONTAIRE POUR RÉALISER UN ESSAI

(30) Priorität: 12.05.2010 DE 102010020395; 20.02.2010 DE 102010008651
(43) Veröffentlichungstag der Anmeldung: 26.12.2012
(73) Patentinhaber: Airbus DS GmbH, 82024 Taufkirchen (DE)
(72) Erfinder: MARZILGER, Robert, 14612 Falkensee (DE); KOLB,Rolf, 88045 Friedrichshafen (DE); KÜBLER, Ulrich, 88677 Markdorf (DE); KERN, Peter, 88626 Salem (DE); JUNG, Steffen, 77815 Brühl (DE); GOLLHOFER, Albert, 79252 Stegen (DE)
(74) Vertreter: Daub, Thomas
(86) Internationale Anmeldenummer: PCT/DE2011/000107
(87) Internationale Veröffentlichungsnummer: WO 2011/100948

(56) Entgegenhaltungen:
- DE-A1-102005 015 715
- US-A- 3 583 322

## Beschreibung

Die Erfindung betrifft eine Vorrichtung zur Fixierung einer Testperson auf einer Standfläche.

Insbesondere betrifft die Erfindung eine Vorrichtung die es ermöglicht, Personen unter Schwerelosigkeit zu fixieren, so dass diese Kräfte auf Ihre Standfläche (Unterstützungsfläche) ausüben können.

Herkömmliche Vorrichtungen basieren auf Fußschlaufen und Seilsysteme. Die Fußschlaufen dienen in der Regel eher der Fortbewegung und Fixierung bei ortsfesten Arbeiten, eine Nutzung für sportliche Tätigkeiten ist jedoch nicht ausgeschlossen. Die Seilsysteme, sind zumeinst im Boden verankert und fixieren die Person, mit Hilfe eines Harnes am Oberkörper, an der Struktur des Weltraumfahrzeuges. Die Fußschlaufen erlauben keine dauerhafte Fixierung. Weiterhin sind sie nicht geeignet eine Versuchsperson mit variabler Kraft auf der Unterstützungsfläche zu fixieren. Die Seilsysteme erlauben zwar eine variable Fixierung der Person bieten jedoch auf Grund Ihrer Anordnung auch große seitliche Führungskräfte.

Standardübungen, wie z.B. ein- oder beidbeiniges Stehen mit geschlossenen Augen, basieren darauf, dass die Testperson über einen definierten Zeitraum möglichst unbewegt stehen beleiben soll, wobei jegliche Bewegung registriert und ausgewertet wird. Solche Übungen sind mit den herkömmlichen Vorrichtungen nur bedingt möglich.

Eine Vorrichtung gemäß den Oberbegriffen der Ansprüche 1 und 4 ist in US 3 583 322 offenbart.

Aufgabe der Erfindung ist es, eine Vorrichtung zur Fixierung einer Testperson auf einer Standfläche anzugeben, bei welcher die seitlichen Führungskräfte reduzieren sind, während weiterhin variable, vertikale Kräfte auf die Versuchsperson einwirken können.

Diese Aufgabe wird mit den Vorrichtungen gemäß Ansprüchen 1 und 4 gelöst. Vorteilhafte Ausführungen sind Gegenstand von Unteransprüchen.

Erfindungsgemäß ist zwischen einem der Testperson angelegten Hüftgurt und einer unterhalb der Standfläche angeordneten Halteplatte mindestens ein Seil gespannt ist.

Die Fixierung ist dabei derart gestaltet, dass sie auf die Testperson eine variable vertikale (Schwerkraft simulierende) Kraft ausüben kann und die dabei auftretenden horizontalen Kräfte minimiert. Durch die Art der Fixierung wird der Person eine maximale Bewegungsfreiheit gewährt, ohne für die Bewegung rückstellende bzw. stützende Kräfte auszuüben, was eine wichtige Anforderung speziell für das koordinative Gleichgewichtstraining darstellt.

Die Erfindung sowie vorteilhafte Ausgestaltungen der Erfindung werden anhand von Figuren näher erläutert. Es zeigen:
- Fig. 1: ein erstes Ausführungsbeispiel,
- Fig. 2: eine erste Ausführungsform der Erfindung,
- Fig. 3: eine zweite Ausführungsform der Erfindung,
- Fig. 4: eine dritte Ausführungsform der Erfindung.

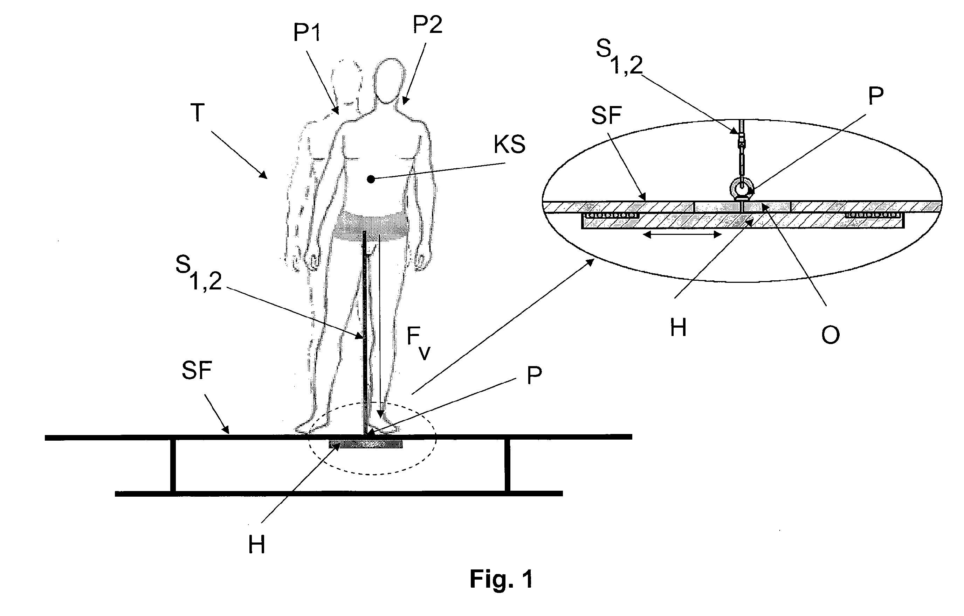

Bei dem, in Fig. 1 dargestellten Ausführungsbeispiel werden zweckmäßig elastisch ausgeführte Seile S zu einem Befestigungspunkt P auf der Halteplatte H zusammengeführt. In Fig. 1 sind beispielhaft zwei Seile ausgeführt, wobei das eine Seil S₁ vorne im Bereich des Bauchs der Testperson T und das andere Seil S₂ hinten im Bereich des Rückens befestigt ist.

Der Befestigungspunkt P ist hierbei zweckmäßig so gewählt, dass er sich in der Ebene befindet, welche durch die Normale auf die Standfläche SF und die Projektion des Körperschwerpunktes KS auf die Standfläche SF aufgespannt wird. Zweckmäßig kann für jedes Seil ein eigener Befestigungspunkt P in der genannten Ebene gewählt werden.

Zweckmäßig weist die Standfläche SF eine Öffnung O auf (vergrößerte Darstellung), welche im Bereich des vertikal auf die Standfläche SF projizierten Schwerpunktes KS der Testperson ausgeführt ist. Die Halteplatte H weist zweckmäßig eine Ausdehnung auf, welche größer ist als die Öffnung O. Die Halteplatte H ist derart unterhalb der Standfläche SF gelagert, dass sie sich in der horizontalen Ebene (Pfeilrichtung) frei bewegen kann. Die Seile S₁ und S₂ sind durch die Öffnung O geführt und verbinden den Hüftgurt der Testperson T mit der Halteplatte H.

Bei einer horizontalen Auslenkung des Hüftgurtes (von Position P1 zu Position P2) der Testperson T folgt die Fixierung am Befestigungspunkt P der Bewegung des Körperschwerpunktes KS, hierdurch wiederum erfahren die elastischen Seile S₁ und S₂ keine Längen- und Kraftänderungen bei Bewegungen in der horizontalen Ebene. Es wirkt in diesem System also nur eine vertikale Kraft F_{V}, die sich aus der Dehnung der Einspannseile S₁ und S₂ ergibt.

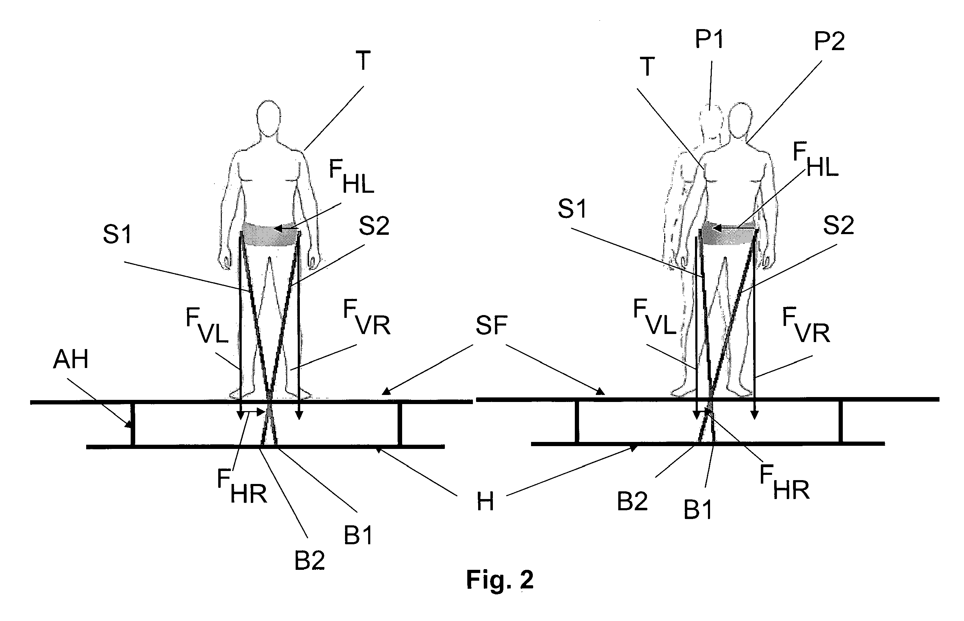

In einer ersten Ausführungsform der Erfindung, dargestellt in Fig. 2, wird die Testperson T beispielhaft mit Hilfe zweier sich überkreuzender elastischer Seile S₁ und S₂ auf der Standfläche SF fixiert. Die Seile S₁ und S₂ sind zur besseren Veranschaulichung jeweils an der Seite am Hüftgurt der Testperson T angebracht. Zweckmäßig können aber auch vier Seile verwendet werden, wobei jeweils ein weiteres Seil im Bereich des Bauches und des Rückens der Testperson T befestigt wird, evtl. auch um 45° verdreht, d.h. lateral aussen vor bzw. hinter der Hüfte (nicht dargestellt).

Die Halteplatte H ist zweckmäßig in einem Abstand d unterhalb der Unterseite der Standfläche SF angeordnet. Die Halteplatte H ist in dieser Ausführungsform nicht verschiebbar, z.B. ist die Halteplatte mit der Unterseite der Standfläche SF fest mittels eines Abstandshalters AH verbunden. Die bezüglich der Öffnung O bei dem ersten Ausführungsbeispiel dargelegten Erläuterungen gelten auch bei dieser Ausführungsform.

Die Befestigungspunkte B₁ und B₂ der elastischen Seile S₁ und S₂ auf der Halteplatte H sind so gewählt, dass sich die Seile S₁ und S₂ im Schnittpunkt SP mit der Standfläche SF kreuzen. Der Vorteil dieser Anordnung liegt in dem sehr geringen Platzbedarf und den sehr kleinen horizontalen Kräften. Zum einen ist durch den relativ großen Winkel, den die Seile S₁ und S₂ mit der Standfläche SF aufspannen, der Kosinusanteil der Zugkraft viel geringer als bei einer Abspannung zu den Seiten, zum anderen bewirkt die X-förmige Anordnung der Seile S₁ und S₂ alternierende horizontale Kräfte F_{HL}. So steigen die horizontalen Rückstellkräfte F_{HL} bei einer Bewegung von Position 1 zu Position 2 zunächst an und erreichen ihr Maximum wenn das Seil S₁ senkrecht zur Standfläche SF steht, danach nimmt die Rückstellkraft wieder ab.

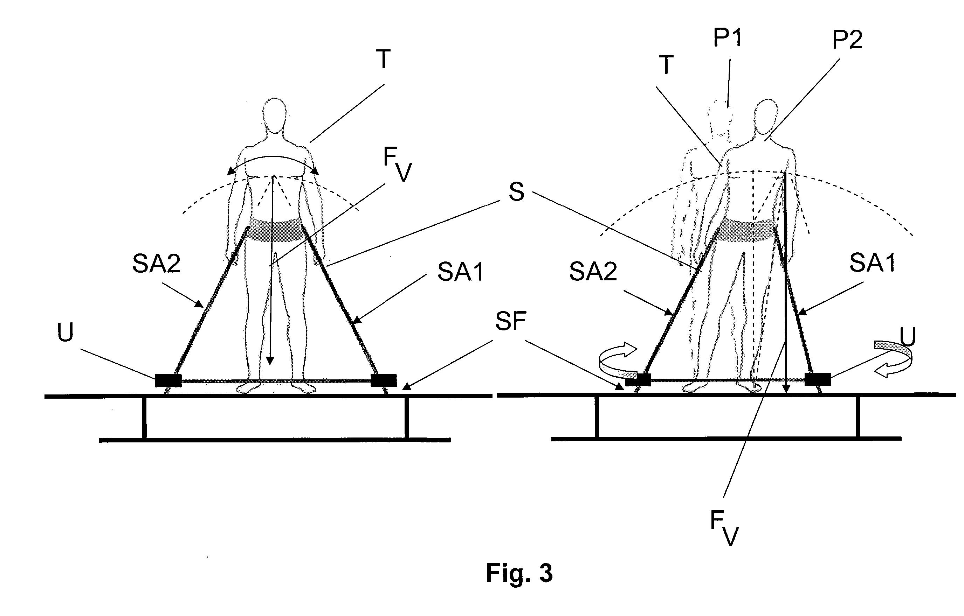

Die zweite Ausführungsform der Erfindung ist in Fig. 3 gezeigt. Auf der Halteplatte H sind mindestens drei Umlenkrollen U (aus zeichnerischen Gründen sind nur 2 Rollen gezeichnet) derart angeordnet sind, dass der Schwerpunkt der von den Umlenkrollen U gebildeten Fläche (nicht dargestellt) mit dem auf die Halteplatte H projizierten Schwerpunkt KS der Testperson T zusammenfällt und ein umlaufendes Seil S von dem der Testperson T angelegten Hüftgurt über die Umlenkrollen U zurück zum Hüftgurt geführt ist.

Das umlaufende Stahlseil S kann mit Hilfe einer Handwinde gespannt werden. Zur Ermittlung der Einspannkraft können Federzugwaagen eingesetzt werden. Durch die konstante Seillänge und unter der Annahme von annährend reibungsfreien Rollen, folgt das Seil stets der Hüftbewegung in der horizontalen Ebene.

Aus Fig. 3 wird deutlich, dass eine Hüftbewegung nach rechts (Position 1 zu 2) zu einem Nachgeben des Seils S auf der rechten Seite führt. Diese Verkürzung des Seilabschnitts SA1 (Abschnitt zwischen Umlenkrolle und Hüftgurt) auf der rechten Seite folgt eine Längung des Seilabschnitts SA2 auf der linken Seite, da das Seil S eine konstante Länge hat. Aufgrund des Nachgebens des Seils auf der einen Seite und der gleichzeitigen Längung des Seils auf der anderen werden die horizontalen Kräfte im System weitestgehend reduziert. Wird die Hüftbewegung als Kreisbahn angenommen, so kommt es auch zu minimalen vertikalen Verschiebungen der Hüfte, diese hat jedoch kaum Einfluss auf das Kräftegleichgewicht im System. Geht man von einer Körperschwankung von ±10° auf der dargestellten Kreisbahn aus, so kommt es bei der dargestellten Lösung zu einer Längendifferenz im Seil von maximal 5mm. Die hierdurch hervorgerufene Reduzierung der vertikalen Kraftkomponente F_{V} ist sehr gering.

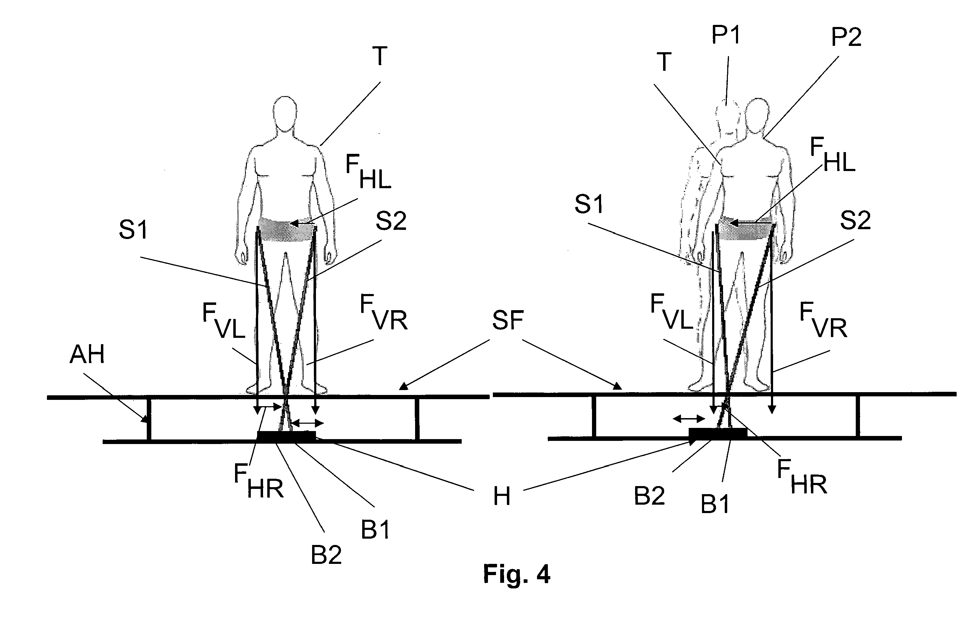

Die dritte Ausführungsform der Erfindung ist in Fig. 4 gezeigt. Bei dieser Ausführungsform handelt es sich um eine Kombination des ersten Ausführungsbeispiels und der ersten Ausführungsform. Die verschiebbare Halteplatte H aus dem ersten Ausführungsbeispiel (Fig.1) ist unterhalb der Standfläche mit einem Abstand AH angeordnet. Hierdurch wird eine Verspannung ähnlich der Kreuzbänder im Kniegelenk erzeugt. Der aufgrund der beschriebenen Anordnung entstandene Roll- Gleit Mechanismus führt dazu, dass die Einspannung in der horizontalen Ebene der Bewegung des Körperschwerpunktes KS folgt und mögliche Kippbewegungen des Körpers gleichzeitig durch die gekreuzten Seile S1, S2 kompensiert werden.

Die erfindungsgemäße Vorrichtung kann vorteilhaft in Schwerelosigkeit, im Wasser oder unter Normalbedingungen verwendet werden.

Mit der erfindungsgemäßen Vorrichtung können aufgrund der Geometrie und Mechanik vertikale Kräfte hervorgerufen werden, mögliche horizontale Kräfte werden jedoch reduziert. Die erzeugten vertikalen Kräfte sind notwendig, um die Person unter Schwerelosigkeit auf der Plattform zu fixieren, bzw. eine Zusatzlast, ähnlich eine Gewichtsweste aufzubringen.

Die Reduzierung der horizontalen Kräfte hat den Sinn, dass eine Stabilisierung der Bewegung durch die Einspannung verhindern wird.

Ziel einer solchen Einspannung ist es nicht, dem Übenden das Üben zu erleichtern, z.B. durch die zusätzliche seitliche Führung, sondern den Schwierigkeitsgrad der Übungsausführung zu erhalten (Schwerelosigkeit) bzw. zu erhöhen (auf der Erde). Das kann durch die zusätzliche vertikale Kraft, die mit Hilfe der Einspannung/ Fixierung aufgebracht wird, erreicht werden.

Bei der vorteilhaften Verwendung der erfindungsgemäßen Vorrichtung im Wasser wird die Auftriebskraft auf einen in Wasser eingetauchten Körper genutzt um ihn quasi schwerelos zu machen. Durch die Verwendung der erfindungsgemäßen Vorrichtung im Wasser, z.B. in einem hierfür vorgesehen Becken, kann für eine Testperson ein Trainingsaufbau realisiert werden, welcher ein Training ermöglicht, bestehend darin, dass die Übungen unter einstellbarer Belastung für den Trainierenden durchgeführt werden können. Die Belastung für den Trainierenden kann einerseits dadurch eingestellt werden, wie tief der Trainierende in das Wasser eintaucht und andererseits durch die Einstellung der zusätzlichen vertikalen Kraft durch die erfindungsgemäße Fixierung des Trainierenden auf der Standfläche.

Die auf den Trainierenden wirkende Kraft ergibt sich dadurch aus der Höhe des Wasserstandes in dem Becken, in welchem sich der Trainierende befindet, und der der Auftriebskraft entgegenwirkenden vertikalen Kraft, mit welcher der Trainierende auf die Standfläche fixiert wird.

Speziell in der Rehabilitation von Verletzungen, z.B. Sportverletzungen kann somit sofort nach der medizinischen Versorgung zum Training wichtiger Bewegungsabläufe, unter zunächst reduzierter Belastung, zurückgekehrt werden. Die durch das Wasser reduzierte Belastung ermöglicht darüber hinaus einen Einsatz der Entwicklung im geriatrischen Bereich oder in der Therapie. Die Belastung kann dann bei Fortschritten im Training wieder erhöht werden.

Die Ausführung des Aufbaus der vorteilhaften Verwendung ist im Prinzip aus anderen Trainingsvarianten wie z.B. dem Laufbandtraining unter Wasser bekannt, jedoch nicht zur Durchführung von sensorimotorischem Training. Die vorteilhafte Verwendung der erfindungsgemäßen Vorrichtung im Wasser stellt damit eine Erweiterung dar, die weitere Vorteile für den Trainierenden bietet.

Neben Rehabilitation, Therapie, Geriatrie und Sport bietet die vorteilhafte Verwendung der erfindungsgemäßen Vorrichtung im Wasser auch eine sehr wirksame Erweiterung der o.g. Verwendung zum Training von Astronauten. Durch die Absolvierung sensorimotorischer Übungen vor einem Raumflug, die dann unter identischen Bedingungen während des Raumfluges wiederholt werden können, wird postuliert, dass die Anpassung an die Schwerelosigkeit, mit den bekannten Problemen wie Koordinationsschwierigkeiten und Raumkrankheit, schneller als mit den bekannten Methoden überwunden werden kann.

Somit kann schon vor Missionsantritt auf der Erde die unterschiedlichen Gravitationsbedingungen eines Zielplaneten simuliert und trainiert werden können. Bereits durchgeführte wissenschaftliche Experimente führen zu der Annahme, dass einmal erlangte sensorimotorische Fähigkeiten durch relativ kurze, regelmäßig durchgeführte Übungen aufrecht erhalten werden können.

## Patentansprüche

1. Vorrichtung zur Fixierung einer Testperson auf einer Standfläche (SF), wobei
zwischen einem der Testperson angelegten Hüftgurt und einer unterhalb der Standfläche angeordneten Halteplatte (H) mindestens zwei Seile (S_{1,2}) gespannt sind und die Standfläche eine, im Bereich des vertikal auf die Standfläche projizierten Schwerpunkts der Testperson ausgeführte Öffnung aufweist, durch welche die mindestens zwei Seile geführt sind,
**dadurch gekennzeichnet, dass**
die Halteplatte in einem Abstand unterhalb der Standfläche angeordnet ist und die Seife zwischen dem Hüftgurt der Testperson und der Halteplatte derart durch die Öffnung geführt sind, dass sich die Seile in der Ebene der Standfläche kreuzen.

2. Vorrichtung nach Anspruch 1,
**dadurch gekennzeichnet, dass**
die Halteplatte horizontal verschiebbar ist.

3. Vorrichtung nach Anspruch 1 oder 2,
**dadurch gekennzeichnet, dass**
die Seile elastisch sind.

4. Vorrichtung zur Fixierung einer Testperson auf einer Standfläche (SF), wobei
zwischen einem der Testperson angelegten Hüftgurt und einer unterhalb der Standfläche angeordneten Halteplatte (H) mindestens ein Steil (S) gespannt ist,
dadurch geftennzeichnet, dass
auf der Halteplatte mindestens drei Umlenkrollen (U) derart angeordnet sind, dass
der Schwerpunkt der von den Umlenkrollen gebildeten Fläche mit dem auf die Halteplatte projizierten Schwerpunkt der Testperson zusammenfällt und ein umlaufendes Seil von dem der Testperson angelegten Hüftgurt über die Umlenkrollen zurück zum Hüftgurt geführt ist.

5. Vorrichtung nach Anspruch 4,
**dadurch gekennzeichnet, dass**
das Seil ein flexibles Stahlseil ist.

6. Verwendung einer Vorrichtung gemäß einem der vorangehenden Ansprüche, in Schwerelosigkeit oder im Wasser.

## Claims

1. Device for fixating a test person on a standing surface (SF),
wherein at least two ropes (S1,2) are tensioned between a hip belt placed on the test person and a retaining plate (H) arranged below the standing surface, and the standing surface has an opening, which is implemented in the region of the centre of gravity of the test person projected onto the standing surface and through which the at least two ropes are guided,
**characterised in that**
the holding plate is arranged at a distance below the standing surface and the ropes are guided, between the hip belt of the test person and the holding plate, through the opening in such a way that the ropes cross in the plane of the standing surface.

2. Device according to claim 1,
**characterised in that**
the holding plate is horizontally displaceable.

3. Device according to claim 1 or 2,
**characterised in that**
the ropes are elastic.

4. Device for fixating a test person on a standing surface (SF),
wherein at least one rope (S) is tensioned between a hip belt placed on the test person and a holding plate (H) arranged below the standing surface,
**characterised in that**
at least three pulleys (U) are arranged on the holding plate in such a way that the centre of gravity of the surface formed by the pulleys coincides with the centre of gravity of the test person projected onto the holding plate and a circumferential rope is guided from the hip belt placed on the test person, via the pulleys, back to the hip belt.

5. Device according to claim 4,
**characterised in that**
the rope is a flexible steel rope.

6. Usage of a device according to one of the preceding claims, in zero-gravity or in water.

## Revendications

1. Dispositif pour fixer un sujet expérimental sur une surface de position (SF),
au moins deux cordes (S_{1,2}) étant tendues entre un sangle de hanches mis au sujet expérimental et une plaque de support (H) disposée au-dessous de la surface de position,
et la surface de position présentant une aperture implémentée dans la région du centre de gravité projeté perpendiculairement sur la surface de position, à travers laquelle les au moins deux cordes sont guidées,
**caractérisé en ce que**
la plaque de support est disposée à une distance au-dessous de la surface de position et les cordes sont guidées, entre le sangle de hanches du sujet expérimental et la plaque de support, dans cette façon que les cordes se croisent dans le plan de la surface de position.

2. Dispositif selon la revendication 1,
**caractérisé en ce que**
la plaque de support est déplaçable horizontalement.

3. Dispositif selon l'une quelconque des revendications 1 ou 2,
**caractérisé en ce que**
les cordes sont élastiques.

4. Dispositif pour fixer un sujet expérimental sur une surface de position (SF), alors qu'au moins une corde (S) est tendue entre un sangle de hanches mis au sujet expérimental et une plaque de support (H) disposée au-dessous de la surface de position,
**caractérisé en ce qu'**
au moins trois poulies (U) sont disposées sur la plaque de support dans cette façon que le centre de gravité de la surface formée par les poulies coïncide avec le centre de gravité du sujet expérimental projeté sur la plaque de support et une corde circonférentielle est guidée du sangle de hanches mis au sujet expérimental via les poulies retour au sangle de hanches.

5. Dispositif selon la revendication 4,
**caractérisé en ce que**
la corde est une corde d'acier flexible.

6. Usage d'un dispositif selon l'un quelconque des revendications précédentes, en apesanteur ou dans l'eau.
